# EUROPÄISCHE PATENTSCHRIFT

(11) **EP 4 429 743 B1**
(45) Veröffentlichungstag und Bekanntmachung des Hinweises auf die Patenterteilung: **30.07.2025**
(21) Anmeldenummer: 21806268.5
(22) Anmeldetag: 09.11.2021
(51) Int. Cl.: A61M 16/08, A61M 16/20

(54) **AUSATEMVENTIL**
EXHALATION VALVE
VALVE D'EXPIRATION

(43) Veröffentlichungstag der Anmeldung: 18.09.2024
(73) Patentinhaber: Löwenstein Medical Technology S.A., 2557 Luxembourg (LU)
(72) Erfinder: HECHLER, Helmut, 91567 Herrieden (DE)
(74) Vertreter: Löwenstein Medical IP
(86) Internationale Anmeldenummer: PCT/EP2021/081151
(87) Internationale Veröffentlichungsnummer: WO 2023/083438

(56) Entgegenhaltungen:
- EP-A1- 1 647 310
- DE-A1- 19 731 068
- US-A- 4 712 580
- US-A1- 2016 256 661

## Beschreibung

Die vorliegende Erfindung betrifft ein Ausatemventil, welches als Steuerventil zur Beatmung von Patienten verwendet wird.

### Technologischer Hintergrund

Ausatemventile der vorliegend interessierenden Art dienen dazu, Atemgas von einem Beatmungsgerät zu empfangen und einem Patienten zuzuführen. Ferner gewährleistet das Ausatemventil, dass das vom Patienten ausgeatmete Atemgas an die Umgebung abgegeben werden kann. Die Ventilfunktion des Ausatemventils steuert somit zwei Prozesse, nämlich das Einatmen sowie das Ausatmen. Die Steuerung wird dadurch vorgenommen, dass eine Membran mit Druckluft beaufschlagt wird, wenn der Patient einatmet, wohingegen keine Druckluftbeaufschlagung erfolgt, wenn der Patient ausatmet. In letzterem Fall kann die vom Patienten ausgeatmete Luft die Membran anheben und über die Auslassöffnung entweichen. Über eine angeschlossene Druckmessleitung kann der Druck in dem Ausatemventil detektiert und im Bedarfsfall für eine Auswertung durch das Beatmungsgerät verwendet werden.

Üblicherweise befindet sich das Ausatemventil sehr nahe am Patienten, beispielsweise in unmittelbarer Nähe zu einer Atemmaske.

### Druckschriftlicher Stand der Technik

Aus der TW 201143828 A1 ist ein Ausatemventil der gattungsgemäßen Art bekannt, bei dem sich die Öffnung für das vom Patienten zurückgeführte ausgeatmete Atemgas auf der der Membran gegenüberliegenden Seite des Ausatemventils befindet. Die Auslassöffnung ist hierbei mit einem Konnektor mit reduziertem

Durchmesser verbunden, um eine Druckdifferenz zwischen Innen- und Außenseite einer Ventilplatte auszubalancieren und Geräusche zu reduzieren.

Die DE 10 2008 026 321 A1 offenbart ein Patientenventil mit einem zweiteilig aufgebauten Grundkörper, wobei der erste Teil des Grundkörpers auf den zweiten Teil aufgesteckt wird. Des Weiteren ist ein dritter Teil vorgesehen, der den Deckel darstellt und mit dem ersten Teil des Grundkörpers über einen Bajonettverschluss verbindbar ist. Die Auslassöffnung für das vom Patienten stammende Atemgas befindet sich im Bereich des Abzweigstutzens.

Aus der EP 1 512 426 A1 ist ein Ausatemventil bekannt, bei dem die Auslassöffnung für vom Patienten stammendes Atemgas durch eine vom Grundkörper zusammen mit dem Deckel gebildete Öffnung gebildet wird. Die Öffnung ist am Ausatemventil in ihrer Position starr vorgegeben.

Aus der EP 3 360 595 B1 ist schließlich ein Ausatemventil bekannt, bei dem der Grundkörper im Bereich des Abzweigstutzens und der Deckel mittels eines Bajonettverschlusses miteinander verbunden werden. Als Auslassöffnung ist ein seitlich am Grundkörper angeformter Auslassstutzen vorgesehen.

Die US 4 712 580 A offenbart eine Ausatmungsventilbaugruppe, die unterschiedliche Druckhaltefähigkeiten bietet und gleichzeitig den Bedarf an Stützelementen und abnehmbaren Ringelementen beseitigt.

### Aufgabe der vorliegenden Erfindung

Die Aufgabe der vorliegenden Erfindung besteht darin, ein verbessertes Ausatemventil zur Verfügung zu stellen.

### Lösung der Aufgabe

Die vorstehende Aufgabe wird durch die Merkmale des Anspruchs 1 gelöst. Zweckmäßige Ausgestaltungen der Erfindung werden in den abhängigen Ansprüchen beansprucht.

Dadurch, dass die Auslassöffnung in der den Ringkanal außenseitig begrenzenden Wand vorgesehen ist und die Auslassöffnung bezüglich der Ausströmrichtung des Atemgases aus der Auslassöffnung bzw. dem Ringkanal relativ zum Grundkörper bzw. dessen Längsachse variierbar ist, kann ohne Positionsveränderung des Ausatemventils am Patienten die Strömungsrichtung des das Ausatemventil verlassenden Atemgases des Patienten bedarfsgerecht variiert werden. Würde sich beispielsweise durch die individuelle Ausrichtung des Ausatemventils relativ zum Patienten der durch das Ausatemgas bedingte Luftstrom beispielsweise gegen den Oberkörper des Patienten richten, kann durch Verstellung der Ausströmrichtung der Auslassöffnung der Luftstrom in eine andere Richtung gelenkt werden. Da sich die Auslassöffnung in dem Ringkanal im Bereich des Abzweigstutzens, vorzugsweise seitlich zum Abzweigstutzen, befindet, kann das Ausatemgas sogleich nach Austritt aus dem Abzweigstutzen das Ausatemventil verlassen, ohne dass es durch Verwirbelungen sowie Druckstaus bewirkende Engstellen am Ausatemventil bzw. dessen Grundkörper durchströmen muss. Hierdurch kann die Strömung des Ausatemgases innerhalb des Ausatemventils so weit wie möglich laminar gehalten werden, was vorteilhaft ist.

Zweckmäßigerweise ist für das Variieren der Auslassöffnung eine Abdeckung vorgesehen, die in ihrer Position entlang des Umfangs des Ringkanals veränderbar, vorzugsweise zur bzw. entlang der den Ringkanal außenseitig begrenzenden Wand verschiebbar ist. Durch Verdrehen bzw. Verschieben der Abdeckung relativ zu der den Ringkanal außenseitig begrenzenden Wand kann somit die Auslassrichtung der Auslassöffnung in einfacher Weise je nach individuellem Bedarf variiert werden.

Gleichzeitig kann der Deckel in einfacher Weise durch eine Klemm- oder Schnappverbindung mit dem Grundkörper verbunden werden. Die Schnappverbindung kann durch einen nach innen gerichteten Vorsprung am Deckel sowie eine komplementäre Rastausnehmung in der Wand (oder umgekehrt) realisiert werden.

Gemäß einer weiteren zweckmäßigen Ausgestaltung weist der Deckel eine Ausnehmung zur Aufnahme des äußeren Teils einer Ventilmembrane auf.

Hierzu kann die Abdeckung mindestens einen Vorsprung, vorzugsweise eine Mehrzahl von Vorsprüngen aufweisen, der bzw. die in die Auslassöffnung eingreift bzw. eingreifen. Die Auslassöffnung bewirkt hierdurch eine Doppelfunktion.

Es ist vorteilhaft, wenn die Abdeckung als Drehring vorgesehen ist. Ein Drehring kann als Kunststoffteil in einfacher Weise im Spritzgießverfahren hergestellt werden und erlaubt zudem eine besonders einfache Montage.

Bei der Abdeckung handelt es sich vorzugsweise um eine solche, die lediglich einen Teil der vollen Winkelmaßlänge eines Kreises aufweist, vorzugsweise eine Winkelmaßlänge im Bereich von 180° bis 270°. Hierdurch kann eine gezielte Ausströmrichtung eingestellt werden. Zudem kann die Abdeckung während der Komplettierung des Ausatemventils in einfacher Weise eingesetzt werden, indem erst geweitet und anschließend auf die Wand aufgeschnappt wird.

Dadurch, dass die Auslassöffnung mehrere entlang des Umfangs des Ringkanals verteilte Langlöcher umfasst, kann der Grundkörper einerseits einfach im Spritzgießverfahren hergestellt werden, andererseits bieten die Langlöcher auch Vorteile in Bezug auf eine verbesserte Ausströmung des Atemgases aus dem Ventil.

Insbesondere können jeweils zwei benachbarte Langlöcher jeweils entlang des Umfangs des Ringkanals lediglich durch einen Steg voneinander getrennt sein. Der Steg kann beidseitig konkav ausgebildete Stegbegrenzungen aufweisen.

Die Abdeckung kann sich vorteilhaft außerhalb der Wand befinden. In diesem Fall ist eine sehr einfache Montage bei gleichzeitiger guter Bedienbarkeit gegeben.

Zweckmäßigerweise erstreckt sich der Ringkanal oder die Letzteren außenseitig begrenzende Wand zumindest im Wesentlichen ausschließlich auf den Bereich des Abzweigstutzens des Grundkörpers und nicht darüber hinaus auf die in Bezug auf die Membran gegenüberliegende Seite des Ausatemventils. Hierdurch werden Engstellen und Verwirbelungen für das vom Patienten stammende Atemgas vermieden.

Zur Steuerung des Ausatemventils ist eine Membran vorgesehen, die unter Druckluftbeaufschlagung das offene Ende des Abzweigstutzens verschließt bzw. bei Beendigung der Druckluftbeaufschlagung und beim Ausatmen des Patienten das ausgeatmete Atemgas ausatemdruck-begründet die Membran anhebt und über das offene Ende des Abzweigstutzens in den Ringkanal gelangen und dort über die Auslassöffnung das Ausatemventil verlassen kann.

Vorzugsweise wird die Membran dadurch fixiert, dass sie auf dem offenen bzw. freien Ende der den Ringkanal außenseitig begrenzenden Wand aufliegt und vom Deckel fixiert, vorzugsweise festgeklemmt, wird. Hierdurch können sowohl der Grundkörper als auch der Deckel konstruktiv einfach ausgestaltet sein. Gleichzeitig ist eine einfache Montage gewährleistet.

Vorzugsweise kann der Deckel zur Fixierung der Membrane eine Ausnehmung aufweisen, in die der äußere Randbereich der Membrane eingreift.

Ferner ist es vorteilhaft, wenn der Deckel der Membrane zusätzlich in Richtung des Steuerdruckanschlusses den erforderlichen Freiraum gibt, so dass sich die Membrane durch den Ausatemdruck heben und das Atemgas ohne nennenswerten zusätzlichem Atemaufwand entweichen kann.

Aufgrund der erfindungsgemäßen Konstruktion kann der Grundkörper vorteilhaft als einstückiges Bauteil hergestellt werden.

### Beschreibung der Erfindung anhand von Ausführungsbeispielen

Eine zweckmäßige Ausgestaltung des erfindungsgemäßen Ausatemventils wird anhand von Zeichnungsfiguren nachstehend näher erläutert. Es zeigen:
- Fig. 1: eine Explosionsdarstellung eines Beispiels eines erfindungsgemäßen Ausatemventils;
- Fig. 2: eine Längsschnittdarstellung durch das Ausatemventil von Fig. 1; sowie
- Fig. 3: eine Schnittdarstellung entlang der Schnittebene A-A aus Fig. 2.

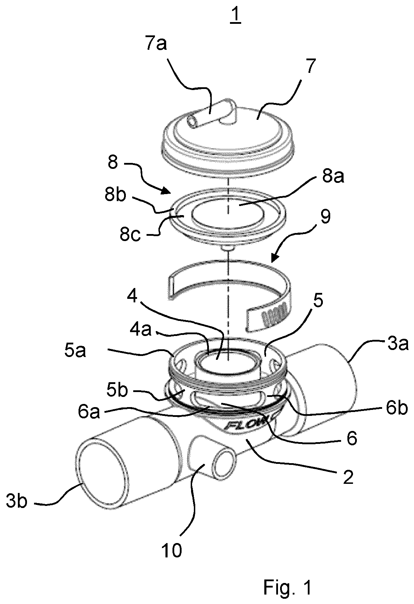

In Fig. 1 ist ein Beispiel eines erfindungsgemäßen Ausatemventils 1 als eine Explosionsdarstellung wiedergegeben. Das Ausatemventil 1 umfasst einen Grundkörper 2, welcher einteilig als Kunststoffteil, vorzugsweise im Spritzgießverfahrenhergestellt, ausgeführt ist. Der Grundkörper 2 umfasst einen Einlass 3a, der dazu vorgesehen ist, Atemgas von einem (in Fig. 1 nicht dargestellten) Beatmungsgerät zu empfangen. Ferner umfasst der Grundkörper 2 einen Auslass 3b, welcher dazu vorgesehen ist, das von dem Beatmungsgerät empfangene Atemgas einem Patienten zuzuführen. Ferner kann der Grundkörper 2 optional einen Anschlussstutzen 10 für einen (nicht dargestellten) Konnektor, an dem eine (in Fig. 1 ebenfalls nicht dargestellte) Druckmessleitung angeschlossen werden kann. Die Druckmessleitung dient dazu, den Druck im Ausatemventil 1 für eine Auswertung durch das Beatmungsgerät zu messen, falls hierzu Bedarf besteht.

Zwischen Einlass 3a und Auslass 3b befindet sich ein oberseitiger Abzweigstutzen 4 mit einem freien Ende 4a. Der Abzweigstutzen 4 wird außenseitig von einer weiteren zylinderförmigen Wand 5b des Grundkörpers 2 umgeben, die mit dem Abzweigstutzen 4 einen Ringkanal 5 bildet. Die Wand 5b erstreckt sich an dem Ausatemventil 1 ausschließlich auf die Bereiche des innerhalb der Wand 5b befindlichen Abzweigstutzens 4. Demzufolge erstreckt sich der Ringkanal 5 zwischen Abzweigstutzen 4 und Wand 5b ausschließlich auf den oberen Bereich des Ausatemventils 1. Der Ringkanal 5 erstreckt sich nicht auf die gegenüberliegende Seite d.h. auf den unteren Bereich des Grundkörpers 2.

In der Wand 5b des Ringkanals 5 ist eine Auslassöffnung 6 vorgesehen, die durch eine Mehrzahl von den Abzweigstutzens 4 konzentrisch umgebende Öffnungen in der Wand 5b gebildet wird. Die Öffnung 6 wird durch eine Mehrzahl von Langlöchern 6a gebildet, die durch jeweils einen Steg 6b voneinander getrennt sind. Die Langlöcher 6a weisen gerundete Endbereiche auf. Sie sind gleich verteilt d. h. symmetrisch entlang des Umfangs des Ringkanals 5 angeordnet.

An der Oberseite des freien Endes 4a des Abzweigstutzens 4 bzw. des freien Endes der Wand 5b befindet sich zur Gewährleistung der Ventilfunktion eine Membrane 8 aus elastischem Material, insbesondere aus Silikon. Die Membrane 8 umfasst einen planen Mittelteil 8a, einen äußeren Randbereich 8b sowie einen gekrümmt verlaufenden Zwischenbereich 8c. Der äußere Bereich 8b befindet sich im komplettierten Zustand des Ausatemventils 1 in einer nach unten orientierten Ausnehmung 7c des Deckels 7.

Der Mittelbereich 8a der Membrane 8 liegt im geschlossenen Zustand des Ausatemventils 1 auf dem freien Ende 4a des Abzweigstutzens 4 auf. Der äußere Randbereich 8b der Membrane 8 liegt am freien Ende der Wand 5b auf. Die Membrane 8 wird gehalten durch den Deckel 7, der auf das obere Ende der Wand 5b aufgeschnappt wird. Hierzu besitzt die Wand eine vorzugsweise umlaufende erste Rastausnehmung 5d, die mit einem entsprechenden Vorsprung 7b am Deckel7 eine Schnapp- oder Rastverbindung gewährleistet.

Aufgrund der Anordnung der Auslassöffnung 6 bzw. der Langlöcher 6a in unmittelbarer Nähe des Abzweigstutzens 4 kann aus dem Abzweigstutzen 4 austretendes Atemgas das Ausatemventil 1 über den Ringkanal 5 durch die Auslassöffnung 6 in einer zumindest im Wesentlichen laminaren Strömung verlassen.

Um die Auslassrichtung des die Auslassöffnung 6 verlassenden Atemgases je nach Bedarf individuell einzustellen, ist zusätzlich eine Abdeckung 9 der Auslassöffnung 6 vorgesehen, die entlang der Wand 5b verschiebbar ist, wodurch durch Verschieben der Abdeckung 9 nur der gewünschte Bereich der Auslassöffnung 6 freigegeben und der weitere Bereich derselben abgedeckt wird.

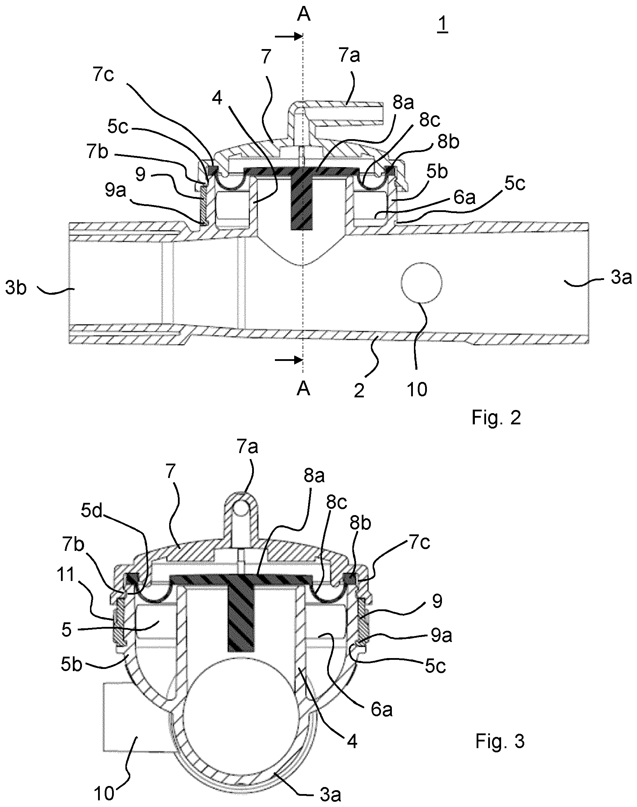

Die Abdeckung 9 ist gemäß Fig. 2 beispielsweise als außenseitig an der Wand 5b anliegender Teilring ausgebildet und beispielsweise mit mindestens einem nach innen gerichteten Vorsprung 9a versehen, der in eine entsprechende außenseitige nutförmige zweite Rastausnehmung 5c der Wand 5b eingreift. Die Anordnung kann auch vertauscht ausgeführt sein. Zusätzlich greift die Oberseite des Teilrings in eine durcheine Stufe 7b am Deckel 7 und der Außenfläche der Wand 5b gebildete Ausnehmung ein. Hierdurch wird der Teilring bzw. die Abdeckung 9 sicher gehalten, gleichzeitig kann der Teilring entlang der Wand 5b verschoben werden.

Die Abdeckung 9 befindet sich an der Außenseite der Wand 5b, unmittelbar an dieser anliegend. Zur Betätigung des Teilrings bzw. der Abdeckung 9 können auf seiner Außenseite Erhebungen 11 oder Rippen eingeformt sein, an denen die Abdeckung 9 gut zugänglich mit der Fingerfläche gefasst und bedarfsgerecht verschoben werden kann. Der Teilring bzw. die Abdeckung 9 deckt vorzugsweise eine Winkelmaßlänge im Bereich von 180° bis 270° ab. Die erfindungsgemäße Konstruktion bieten den Vorteil, dass das erfindungsgemäße Ausatemventil 1 eine verstellbare Auslassöffnung 6 aufweist, sich jedoch dennoch durch eine einfache Konstruktion auszeichnet und einfach komplettiert, d.h. montiert, werden kann.

Fig. 2 zeigt das Ausatemventil 1 der Fig. 1 beispielhaft in geschlossenem Zustand, bei dem die Membrane 8 mit ihrem Mitteilteil 8a am freien Ende 4a des Abzweigstutzens 4 aufliegt und mittels Druckluft über den Anschluss 7a auf das freie Ende 4a gedrückt, um einen leckagefreien Verschluss des Abzweigstutzens 4 bei anliegendem Steuerdruck sicherzustellen. In dieser Stellung kann Atemgas von der Atemgaskonditioniereinrichtung über das Ausatemventil 1 hin zum Patienten strömen, ohne dass es über den Abzweigstutzen 4 entweichen kann.

Wenn der Patient ausatmet, muss das Ausatemventil 1 geöffnet werden. Für das Öffnen des Ausatemventils 1 wird die Druckluftzufuhr am Anschluss 7a aufgehoben, sodass bei einem Einstellen eines Drucks aufgrund der Ausatemtätigkeit des Patienten der Mittelteil 8a der Membrane 8 angehoben wird und Ausatemgaszuerst in den Ringkanal 5 und von dort über die Auslassöffnung 6 ins Freie entweichen kann. Die Bewegung des Mittelteils 8a der Membrane 8 wird hierbei durch den gekrümmt verlaufenden Zwischenbereich 8c der Membrane 8 ermöglicht, der lediglich eine sehr dünne Schichtdicke aufweist, sodass der Mittelteil 8a der Membrane 8 bereits bei geringem Druck angehoben werden kann. Für die Steuerung des Beatmungsgerätes kann der Druck über den Anschlussstutzen 10 gemessen werden, falls eine solche Möglichkeit beim Beatmungsgerät vorgesehen ist. Das Beatmungsgerät stellt das Atemgas entsprechend einer Beatmungskurve bereit, wertet optional die verfügbare Druckmessleitung aus und nimmt die Ventilsteuerung vor. Dies erfolgt über die (nicht dargestellte) Druckmessleitung, die am Anschlussstutzen 10 angeschlossen wird. Für Beatmungsgeräte ohne diese Möglichkeit kann bei dem erfindungsgemäßen Ausatemventil 1 der Anschlussstutzen 10 ersatzlos entfallen.

Aus Fig. 2 sowie Fig. 3 ist jeweils ersichtlich, dass der Deckel 7 mittels des Vorsprungs 7b auf die Wand 5b aufgrund der dortigen ersten Rastausnehmung 5d in einfacher Weise aufgeschnappt werden kann und die Membrane 8 hierdurch sicher in Position gehalten werden kann.

Wie aus Fig. 2 ebenfalls hervorgeht, ist die eine Seite des Ringkanals 5 mittels der Abdeckung 9 verdeckt, sodass lediglich über einen Teilbereich der Auslassöffnung 6 Ausatemluft entweichen kann. Bei dem verschlossenen Bereich der Auslassöffnung 6 handelt es sich in Fig. 2 um den Bereich, der dem Auslass 3b zugeordnet ist. Der dem Einlass 3a zugeordnete Bereich der Auslassöffnung 6 ist demgegenüber offen, wie dies auch aus Fig. 2 sowie Fig. 3 ersichtlich ist.

Die vorliegende Erfindung gewährleistet ein einfach herzustellendes Ausatemventil mit sehr günstigen Strömungsverhältnissen in Bezug auf das vom Patienten ausgeatmete Atemgas bei gleichzeitiger Möglichkeit, die Auslassrichtung des Atemgases bedarfsgerecht zu variieren.

### BEZUGSZEICHENLISTE

- 1: Ausatemventil
- 2: Grundkörper
- 3: Durchlasskanal
- 3a: Einlass
- 3b: Auslass
- 4: Abzweigstutzen
- 4a: freies Ende des Abzweigstutzens
- 5: Ringkanal
- 5a: offenes Ende des Ringkanals
- 5b: Wand
- 5c: zweite Rastausnehmung
- 5d: erste Rastausnehmung
- 6: Auslassöffnung
- 6a: Langloch
- 6b: Steg
- 7: Deckel
- 7a: Anschluss für Druckleitung
- 7b: Vorsprung am Deckel
- 7c: Ausnehmung
- 8: Membrane
- 8a: Mittelteil der Membrane
- 8b: äußerer Randbereich der Membrane
- 8c: gekrümmt verlaufender Zwischenbereich der Membrane
- 9: Abdeckung
- 9a: Vorsprung
- 10: Anschlussstutzen
- 11: Erhebung

## Patentansprüche

1. Ausatemventil umfassend:
einen Grundkörper (2), wobei der Grundkörper folgendes umfasst:
einen hohlen Durchlasskanal (3) mit einem Einlass (3a), einem Auslass (3b), wobei der Einlass (3a) dafür vorgesehen ist, Atemgas von einem Beatmungsgerät zu empfangen und der Auslass (3b) dafür vorgesehen ist, das von dem Beatmungsgerät empfangene Atemgas einem Patienten zuzuführen,
einen Abzweigstutzen (4), der zwischen Einlass (3a) und Auslass (3b) angeordnet ist und ein freies Ende (4a) aufweist,
einen außenseitig durch eine Wand (5b) begrenzten, den Abzweigstutzen (4) vorzugsweise konzentrisch, umgebenden Ringkanal (5), der ebenfalls ein freies Ende (5a) aufweist,
mindestens eine Auslassöffnung (6) für vom Patienten ausgeatmetes, über den Auslass (3b) wieder zurückgeführtes und über den Abzweigstutzen (4) abgezweigtes Atemgas,
einen Deckel (7) mit einem Anschluss (7a) für den Anschluss einer Druckluft-Steuerleitung,
eine Membrane (8), die an dem jeweils freien Ende (4a, 5a) des Abzweigstutzens (4) bzw. der Wand (5b) angeordnet ist und es in Anhängigkeit des an der dem jeweils offenen Ende (4a, 5a) abgewandten Seite der Membrane (8) anstehenden Drucks ermöglicht, dass vom Patienten ausgeatmete über den Abzweigstutzen (4) abgezweigte Atemgas in den Ringkanal (5) gelangen kann, **dadurch gekennzeichnet, dass**
die Auslassöffnung (6) in der Wand (5b) vorgesehen ist und die Auslassöffnung (6) bezüglich der Ausströmrichtung des Atemgases aus der Auslassöffnung (6) variierbar ist.

2. Ausatemventil nach Anspruch 1, **dadurch gekennzeichnet, dass** eine Abdeckung (9) vorgesehen ist, die in ihrer Position entlang des Umfangs des Ringkanals (5) veränderbar, vorzugsweise zum Ringkanal (5) bzw. entlang der Wand (5b) verschiebbar, ist.

3. Ausatemventil nach Anspruch 2, **dadurch gekennzeichnet, dass** die Abdeckung (9) durch eine Stufe (7b) im Deckel (7) und/oder eine Ausnehmung (5c) in der Wand (5b) geführt ist.

4. Ausatemventil nach Anspruch 3, **dadurch gekennzeichnet, dass** die Abdeckung (9) an der Außenseite der Wand (5b) angeordnet ist.

5. Ausatemventil nach den Ansprüchen 2 bis 4, **dadurch gekennzeichnet, dass** als Abdeckung (9) ein Drehring vorgesehen ist.

6. Ausatemventil nach den Ansprüchen 2 bis 5, **dadurch gekennzeichnet, dass** die Abdeckung (9) eine Winkelmaßlänge im Bereich von 180° bis270° aufweist.

7. Ausatemventil nach den vorhergehenden Ansprüchen, **dadurch gekennzeichnet, dass** die Auslassöffnung (6) mehrere entlang des Umfangs des Ringkanals (5), vorzugsweise gleichmäßig, verteilte Langlöcher (6a) umfasst.

8. Ausatemventil nach Anspruch 7, **dadurch gekennzeichnet, dass** jeweils zwei benachbarte Langlöcher (6a) durch einen Steg (6b) voneinander getrennt sind.

9. Ausatemventil nach Anspruch 7 oder 8, **dadurch gekennzeichnet, dass** die Auslassöffnung vier, vorzugsweise symmetrisch entlang des Umfangs angeordnete, Langlöcher (6a) umfasst.

10. Ausatemventil nach den vorhergehenden Ansprüchen, **dadurch gekennzeichnet, dass** sich der Ringkanal (5) oder sich die Wand (5b) zumindest im Wesentlichen ausschließlich auf den Bereich des Abzweigstutzens (4) erstreckt.

11. Ausatemventil nach den vorhergehenden Ansprüchen, **dadurch gekennzeichnet, dass** die Membrane (8) unter Druckluft-Beaufschlagung das offene Ende (4a) des Abzweigstutzens (4) verschließt.

12. Ausatemventil nach den vorhergehenden Ansprüchen, **dadurch gekennzeichnet, dass** die Membrane (8) auf dem offenen Ende der Wand (5b) aufliegt und vom Deckel (7) fixiert wird.

13. Ausatemventil nach den vorhergehenden Ansprüchen, **dadurch gekennzeichnet, dass** der Deckel (7) zur Fixierung der Membrane (8) eine Ausnehmung (7c) aufweist.

14. Ausatemventil nach den vorhergehenden Ansprüchen, **dadurch gekennzeichnet, dass** der Grundkörper (2) einstückig ausgebildet ist.

## Claims

1. An exhalation valve comprising:
a base body (2), wherein the base body comprises the following:
a hollow through-channel (3) with an inlet (3a), an outlet (3b), wherein the inlet (3a) is provided to receive respiratory gas from a ventilator and the outlet (3b) is provided to supply the respiratory gas received from the ventilator to a patient,
a branch nozzle (4), which is arranged between the inlet (3a) and the outlet (3b) and has a free end (4a),
an annular channel (5), which is delimited to the outside by a wall (5b), surrounds the branch nozzle (4) preferably concentrically, and also has a free end (5a),
at least one outlet opening (6) for respiratory gas that is exhaled by the patient, fed back via the outlet (3b), and branched off via the branch nozzle (4),
a lid (7) with a connection (7a) for connecting a compressed air control line,
a membrane (8), which is arranged on the free ends (4a, 5a) of the branch nozzle (4) and the wall (5b), respectively, and, depending on the pressure applied to the membrane (8) on the side facing away from open ends (4a, 5a), makes it possible for respiratory gas exhaled by the patient and branched off via the branch nozzle (4) to enter the annular channel (5), **characterized in that**
the outlet opening (6) is provided in the wall (5b) and the outlet opening (6) is variable with regard to the outflow direction of the respiratory gas out of the outlet opening (6).

2. The exhalation valve according to claim 1, **characterized in that** a cover (9) is provided, the position of which along the circumference of the annular channel (5) is modifiable, preferably displaceable in relation to the annular channel (5) or, respectively, along the wall (5b).

3. The exhalation valve according to claim 2, **characterized in that** the cover (9) is guided by a step (7b) in the lid (7) and/or a recess (5c) in the wall (5b).

4. The exhalation valve according to claim 3, **characterized in that** the cover (9) is arranged on the outside of the wall (5b).

5. The exhalation valve according to claims 2 to 4, **characterized in that** a rotating ring is provided as the cover (9).

6. The exhalation valve according to claims 2 to 5, **characterized in that** the cover (9) has an angular measurement length in the range from 180° to 270°.

7. The exhalation valve according to the preceding claims, **characterized in that** the outlet opening (6) comprises multiple elongated holes (6a) distributed, preferably evenly, along the circumference of the annular channel (5).

8. The exhalation valve according to claim 7, **characterized in that** each two adjacent elongated holes (6a) are separated from each other by a rib (6b).

9. The exhalation valve according to claim 7 or 8, **characterized in that** the outlet opening comprises four elongated holes (6a), preferably arranged symmetrically along the circumference.

10. The exhalation valve according to the preceding claims, **characterized in that** the annular channel (5) or the wall (5b) extends at least substantially exclusively on the region of the branch nozzle (4).

11. The exhalation valve according to the preceding claims, **characterized in that** the membrane (8) closes the open end (4a) of the branch nozzle (4) when compressed air is applied.

12. The exhalation valve according to the preceding claims, **characterized in that** the membrane (8) lies on the open end of the wall (5b) and is fixed in place by the lid (7).

13. The exhalation valve according to the preceding claims, **characterized in that** the lid (7) has a recess (7c) for fixing the membrane (8) in place.

14. The exhalation valve according to preceding claims, **characterized in that** the base body (2) is designed as a single piece.

## Revendications

1. Valve d'expiration comportant :
un corps de base (2), dans laquelle le corps de base comporte les éléments suivants :
un canal de passage creux (3) doté d'une entrée (3a), d'une sortie (3b), dans laquelle l'entrée (3a) est destinée à recevoir du gaz respiratoire à partir d'un appareil respiratoire et la sortie (3b) est destinée à acheminer le gaz respiratoire reçu depuis l'appareil respiratoire vers un patient,
un embout de dérivation (4) disposé entre l'entrée (3a) et la sortie (3b) et présentant une extrémité libre (4a),
un canal annulaire (5) délimité par une paroi (5b) du côté extérieur et entourant l'embout de dérivation (4), de préférence de façon concentrique, lequel présente également une extrémité libre (5a),
au moins une ouverture de sortie (6) pour du gaz respiratoire expiré par le patient, renvoyé par le biais de la sortie (3b) et dérivé par le biais de l'embout de dérivation (4),
un couvercle (7) doté d'un raccord (7a) pour le raccordement d'une conduite de commande d'air comprimé,
une membrane (8) disposée à l'extrémité respectivement libre (4a, 5a) de l'embout de dérivation (4) ou de la paroi (5b) et permettant à du gaz respiratoire expiré par le patient, dérivé par le biais de l'embout de dérivation (4), de parvenir dans le canal annulaire (5), en fonction de la pression régnant du côté de la membrane (8) opposé à l'extrémité respectivement ouverte (4a, 5a), **caractérisée en ce que** l'ouverture de sortie (6) est prévue dans la paroi (5b) et l'ouverture de sortie (6) est modulable quant à la direction d'écoulement du gaz respiratoire hors de l'ouverture de sortie (6).

2. Valve d'expiration selon la revendication 1, **caractérisée en ce qu'**il est prévu un recouvrement (9), lequel est modifiable dans sa position le long de la circonférence du canal annulaire (5), de préférence déplaçable vers le canal annulaire (5) ou le long de la paroi (5b).

3. Valve d'expiration selon la revendication 2, **caractérisée en ce que** le recouvrement (9) est guidé par un gradin (7b) dans le couvercle (7) et/ou une cavité (5c) dans la paroi (5b).

4. Valve d'expiration selon la revendication 3, **caractérisée en ce que** le recouvrement (9) est disposé sur le côté extérieur de la paroi (5b).

5. Valve d'expiration selon les revendications 2 à 4, **caractérisée en ce qu'**un anneau rotatif est prévu en tant que recouvrement (9).

6. Valve d'expiration selon les revendications 2 à 5, **caractérisée en ce que** le recouvrement (9) présente une longueur de mesure angulaire dans la plage de 180° à 270°.

7. Valve d'expiration selon l'une des revendications précédentes, **caractérisée en ce que** l'ouverture de sortie (6) comporte plusieurs trous oblongs (6a) répartis le long de la circonférence du canal annulaire (5), de préférence de façon régulière.

8. Valve d'expiration selon la revendication 7, **caractérisée en ce que** deux trous oblongs (6a) adjacents sont respectivement séparés l'un de l'autre par une âme (6b).

9. Valve d'expiration selon la revendication 7 ou 8, **caractérisée en ce que** l'ouverture de sortie comporte quatre trous oblongs (6a), disposés de préférence symétriquement le long de la circonférence.

10. Valve d'expiration selon les revendications précédentes, **caractérisé en ce que** le canal annulaire (5) ou la paroi (5b) s'étend au moins essentiellement exclusivement sur la région de l'embout de dérivation (4).

11. Valve d'expiration selon les revendications précédentes, **caractérisée en ce que** la membrane (8) ferme l'extrémité ouverte (4a) de l'embout de dérivation (4) par application d'air comprimé.

12. Valve d'expiration selon les revendications précédentes, **caractérisée en ce que** la membrane (8) repose sur l'extrémité ouverte de la paroi (5b) et est fixée par le couvercle (7).

13. Valve d'expiration selon les revendications précédentes, **caractérisée en ce que** le couvercle (7) présente une cavité (7c) pour la fixation de la membrane (8).

14. Valve d'expiration selon les revendications précédentes, **caractérisée en ce que** le corps de base (2) est réalisé d'un seul tenant.
